# NEUE EUROPÄISCHE PATENTSCHRIFT

(11) **EP 1 297 187 B2**
(45) Veröffentlichungstag und Bekanntmachung des Hinweises auf die Entscheidung über den Einspruch: **13.08.2014**
(45) Hinweis auf die Patenterteilung: 30.01.2008
(21) Anmeldenummer: 01956475.6
(22) Anmeldetag: 15.06.2001
(51) Int. Cl.: C12Q 1/70, G01N 33/569, C12Q 1/68

(54) **Verfahren zum Nachweis von Influenza A/B-Viren in Speichel**
Method for the detection of Influenza A/B viruses in saliva
Procédé de dépistage des virus grippeaux A/B dans la salive

(30) Priorität: 15.06.2000 DE 10028837
(43) Veröffentlichungstag der Anmeldung: 02.04.2003
(73) Patentinhaber: Roche Diagnostics GmbH, 68305 Mannheim (DE); F.Hoffmann-La Roche AG, 4070 Basel (CH)
(72) Erfinder: KLEPP, Juergen, 76676 Graben-Neudorf (DE); SCHLIPFENBACHER, Reiner, 67098 Duerkheim (DE)
(74) Vertreter: Dick, Alexander
(86) Internationale Anmeldenummer: PCT/EP2001/006735
(87) Internationale Veröffentlichungsnummer: WO 2001/096595

(56) Entgegenhaltungen:
- EP-A- 1 010 981
- WO-A-92/12256
- FR-A- 2 708 348
- US-A- 5 714 341
- US-A- 6 015 664
- COVALCIUC KA, WEBB KH & CARLSON CA: JOURNAL OF CLINICAL MICROBIOLOGY, Bd. 37, Nr. 12, Dezember 1999 (1999-12), Seiten 3971-3974, XP002186401
- ATMAR RL, BAXTER BD, DOMINGUEZ EA & TABER LH: JOURNAL OF CLINICAL MICROBIOLOGY, Bd. 34, Nr. 10, Oktober 1996 (1996-10), Seiten 2604-2606, XP002186402

## Beschreibung

Die Erfindung betrifft ein Verfahren für den Nachweis von Influenza A/B-Viren sowie die Verwendung von Speichel als Probenmaterial für den Nachweis von Influenza A/B-Viren.

Grippe ist eine häufig unterschätzte Infektionskrankheit, die insbesondere bei älteren Menschen und bei Risikopatienten zu einer hohen Morbiditäts- und Mortalitätsrate führen kann. Influenza A- und/oder Influenza B-Viren (im Folgenden auch kurz: Influenza A/B-Viren) sind verantwortlich für die echte Virusgrippe, an der weltweit jährlich mehrere 100 Millionen Menschen erkranken. Die Influenza A- und B-Viren befallen dabei primär den Nasen-, Mund- und Rachenraum und verursachen beim Betroffenen zunächst allgemein respiratorische Symptome.

Selbst dem erfahrenen Mediziner ist es nicht möglich, ausschließlich aufgrund der klinischen Symptomatik des Patienten die Grippe mit hoher Sicherheit zu diagnostizieren, da auch andere, den Nasen- oder Rachenraum befallende Viren wie etwa Adenoviren, Parainfluenzaviren oder Respiratory Syncitial-Viren (RS-Viren) eine vergleichbare Symptomatik verursachen.

Aufgrund der hohen medizinischen Bedeutung der Influenzainfektion (Grippe) verfügt inzwischen nahezu jedes Land der Erde über ein national organisiertes Grippeüberwachungssystem. Dabei werden von niedergelassenen Ärzten Verdachtspatienten Nasen- und/oder Rachenabstriche entnommen und diese an das jeweilige nationale Referenzzentrum eingeschickt. Der Nachweis der Influenza A/B-Viren erfolgt dann üblicherweise durch Elution des Abstrichtupfers und anschließender Kultivierung der Patientenproben auf Säugetierzellen, beispielsweise MDCK-Zellen (Madin-Darby Canine Kidney-Zellen).

Die Kultivierung in diesen Speziallabors kann bis zu 14 Tage in Anspruch nehmen und ist daher als Diagnose für den einzelnen Patienten nicht von unmittelbarer Relevanz. Vielmehr besteht die Aufgabe der nationalen Referenzzentren darin, die kultivierten Viren zu typisieren und zu subtypisieren, und die Ergebnisse an die Weltgesundheitsorganisation (WHO) zu melden. Aufgabe der Impfstoffhersteller ist es dann, aufgrund der jährlichen Empfehlung der WHO die nächstjährigen Grippeimpfstoffe an die zuletzt zirkulierenden Virusstämme anzupassen.

Der Grund der hohen genetischen und damit verbundenen immunologischen Variabilität, insbesondere bei Influenza A-Viren, liegt darin, daß es neben der gewöhnlichen genetischen Drift (Punktmutation) in seltenen Fällen auch zu einem genetischen Shift ("Reassortment", d.h. Neusortierung der Virusgene) kommen kann. Dies wird bedingt durch die Tatsache, daß das Genom der Influenza-Viren im Gegensatz zu anderen Viren segmentiert vorliegt und daß Influenza A sowohl human- als auch tierpathogen ist.

Die an der Oberfläche des Virus sitzenden immundominanten Antigene sind das Hämagglutinin (H) und die Neuraminidase (N). Bei Influenza A sind derzeit 15 Subtypen des Hämagglutinins (H1 - H15) und 9 Subtypen der Neuraminidase (N1 - N9) bekannt.

Beispielsweise kann es bei der Coinfektion eines Wirtes (z. B. eines Schweines) mit einem humanpathogenen Influenzavirus vom A-Typ und einem Influenza A-Vogelvirus zu einer Neusortierung ("Reassortment") des Virusgenoms und damit zu einem neuen Influenza A-Virus-Subtyp kommen, der dann bei Rückübertragung auf den Menschen dessen Immunsystem vollkommen unterläuft. Jüngstes Beispiel dafür war das Auftreten der sogenannten Vogelvirusgrippe im Mai 1997 in Hong Kong (Typ A/H5N1/Hong Kong/156/97), bei der trotz frühzeitigen Erkennens und intensivmedizinischer Behandlung 6 der 18 erkrankten Patienten verstarben.

Um jedoch zu einer größeren Epidemie oder sogar zu einer weltweiten Pandemie zu führen, muß ein neuer Influenza-Virus-Subtyp direkt von Mensch zu Mensch übertragbar sein, wie es etwa 1918/19 bei dem erstmaligen Auftreten des Subtyps H1N1 (Spanische Grippe, weltweit ca. 50 Millionen Tote), 1957 (H2N2, Asiatische Grippe, ca. 1 Million Opfer) und 1968 (H3N2 Hong Kong Grippe, ca. 1 Million Opfer) der Fall war.

Seit kurzem ist eine neue Generation von Grippemitteln verfügbar, die sogenannten Neuraminidaseinhibitoren, die erstmals eine kausale Therapie der Grippe ermöglichen und daher in Expertenkreisen als echter Durchbruch in der Grippetherapie angesehen werden. Klinische Studien bei der Zulassung dieser neuen Substanzklasse haben gezeigt, daß der Therapieerfolg maßgeblich von einem frühen Therapiebeginn nach Auftreten der ersten klinischen Symptome abhängt. Aufgrund dieser neuen therapeutischen Option, der Notwendigkeit eines frühen Therapiebeginns einerseits und der relativ unspezifischen klinischen Symptomatik andererseits erscheint eine schnelle Individualdiagnose als Therapieentscheidung hilfreich.

Daher haben vor kurzem einige Diagnostikahersteller immunologische Grippeschnellteste auf Basis eines Antigennachweises entwickelt, wobei diese Schnellteste auf Nasen- und/oder Rachenabstriche bzw. auf durch Nasenspülungen gewonnene Flüssigkeit als Probenmaterial zurückgreifen. Beispiele für solche Grippeschnellteste sind der Influenza A/B Rapid Test der Firma Roche Diagnostics GmbH, Quick Vue von Quidel und AB FLU OIA von Biostar.

Ein besonderes Problem bei der Probengewinnung besteht darin, aus dem befallenen Bereich des Nasen- und Rachenraumes Probenmaterial zu entnehmen, das für einen Nachweis ausreichende Mengen an Influenza A- und/oder Influenza B-Viren (in Folgenden kurz: Influenza A/B-Viren) enthält. Die Qualität der Probennahme hat somit einen unmittelbaren Einfluß auf die Positivitätsrate der Schnellteste, deren klinische Sensitivität üblicherweise bei etwa 70 % liegt, d. h. in 70 % aller durch die Referenzmethode (Zellkultur) als Influenza-positiv beurteilten Proben ist auch der immunologische Schnelltest positiv. Die Diagnostikahersteller weisen daher in den Beipackzetteln ihrer Influenza-Schnellteste darauf hin, daß die Probennahme nur von speziell geschultem medizinischen Personal durchgeführt werden sollte, um zu gewährleisten, dass das gewonnene Probenmaterial tatsächlich ausreichende Mengen an Influenza A/B-Viren enthält.

Desweiteren kommt erschwerend hinzu, daß die im Rachenraum abzustreichenden Bezirke (Rachenhinterwand, Pharynx, Tonsillen) sich je nach vorliegendem Virusinfekt durchaus unterscheiden können. Dies bedeutet zum Beispiel, daß zum Nachweis einer Streptokokkeninfektion andere Rachenbezirke abgestrichen werden müssen als etwa zum Nachweis von Influenza A/B-Viren.

Die Entnahme eines Nasen-/Rachenabstriches ist für den Patienten unangenehm und insbesondere bei (kleinen) Kindern problematisch. Kinder sind andererseits zumindest in der Frühphase einer Influenzaepidemie aufgrund ihrer sozialen Kontakte (Kindergarten, Schule) und des oftmals noch nicht voll ausgeprägten Immunsystems die Hauptüberträger der Virusgrippe.

Da Nasen- und Rachenabstriche kein homogenes Probenmaterial darstellen, wird die Positivitätsrate eines Influenza-Nachweises neben der Qualität des Abstriches auch durch die korrekte Elution, d.h. Überführung des Abstrichmaterials in die Flüssigphase, die dem nachgeschalteten Test als eigentliches "Probenmaterial" dient, mitbestimmt.

Insbesondere aufgrund der neuen therapeutischen Option (Neuraminidaseinhibitoren) wird zukünftig ein stark zunehmender Bedarf am Nachweis von Grippeviren (Influenza A/B-Viren) beim niedergelassenen Arzt oder vom Patienten selbst vorliegen, der bei dem bisher verwendeten Probenmaterial (Abstrich, Nasenspülung) mit der Notwendigkeit der Probennahme durch geschultes (medizinisches) Personal nicht in Einklang zu bringen ist.

Covalciuc et al., Journal of Clinical Microbiology 37 (1999) 3971 - 3974, vergleichen die Eignung von vier unterschiedlichen Probenmaterialien (Nasal aspirate, Nasopharyngeal swab, Throat swab, Sputum) für den Nachweis von Influenza A und B Viren mittels optischer Immunoassays. Je nach verwendetem Probenmaterial ergeben sich dabei unterschiedliche Sensitivität, Spezifität und Vergleichbarkeit mit Viruskulturbestimmungen.

WO 92/12256 schlägt Verfahren und Testkits zum Nachweis von Virusinfektionen vor. Zum Nachweis von Atemwegsinfekten werden als mögliche Probenmaterialien "nasal apsirate", "nasal washes", "pharyngeal swabs", "nasopharyngeal swabs", "throat washings", "throat swabs", "nasopharyngeal aspirates", "tracheal aspirates" und "bronchial aspirates" genannt. Für den Nachweis von Influenza werden ausschließlich "throat swabs" oder "throat washings" empfohlen.

Die Aufgabe der vorliegenden Erfindung ist es, die Nachteile des Standes der Technik zu beseitigen. Insbesondere besteht die der Erfindung zugrunde liegende Aufgabe darin, ein Verfahren zum Nachweis einer Influenza A- und/oder Influenza B-Virus-Infektion bereitzustellen, das von ungeschultem Personal oder idealerweise dem Patienten selbst einfach durchzuführen ist. Vor allem ist Aufgabe der Erfindung, ein von ungeschultem Personal oder idealerweise dem Patienten selbst einfach und problemlos zu gewinnendes, idealerweise homogenes Probenmaterial zu finden, aus dem zuverlässlich Influenza A- und/oder Influenza B-Viren nachgewiesen werden können.

Diese Aufgabe wird durch den Gegenstand der Erfindung, wie er in den unabhängigen Patentansprüchen charakterisiert ist, gelöst. Bevorzugte Ausführungsformen der Erfindung sind in den abhängigen Ansprüchen angegeben.

Gegenstand der Erfindung ist ein Verfahren zum Nachweis einer Infektion mit dem Influenza A- und/oder Influenza B-Virus umfassend die Schritte
i) Gewinnung einer Speichelprobe des zu untersuchenden Individuums
ii) Vorbereitung der Speichelprobe für den Nachweis bzw. eine Nachweisreaktion; und
iii) Nachweis des Influenza A- und/oder Influenza B-Virus in der Speichelprobe, wobei die Speichelprobe spontan gebildeter Speichel ist, wie weiter unten definiert.

Schließlich ist Gegenstand der Erfindung die Verwendung von spontan gebildetem Speichel als Probenmaterial zum Nachweis einer Infektion mit dem Influenza A- und/oder Influenza B-Virus wie in Anspruch 6 beschrieben.

Unerwarteterweise wurde gefunden, daß aus spontan gebildetem Speichel als Probenmaterial mit etablierten diagnostischen Verfahren ein zuverlässiger Nachweis von Influenza A/B-Viren möglich ist.

Insbesondere erstaunlich ist, dass der Nachweis von Influenza A/B-Viren aus Speichel möglich ist, ohne dass die Viren bei der Probennahme im Speichel angereichert werden müssen, sondern dass vielmehr spontan gebildeter Speichel als Probenmaterial für den Nachweis von Influenza A/B-Viren genügt. Unter "spontan gebildeter Speichel" soll für die vorliegende Erfindung verstanden werden, dass für den Nachweis von Influenza A/B-Viren bei der Probennahme der Speichelprobe keine Anreicherung der Influenza A/B-Viren in der Probe erfolgt. Es wird lediglich der sich spontan im Mundraum befindliche Speichel gesammelt und beispielsweise in ein Speichelsammelgefäß überführt (z. B. durch Spucken o. ä.). Es ist auch möglich, die Speichelprobe mit Hilfe eines saugfähigen Materials, beispielsweise eines Baumwollvlieses (Tupfer), im Mundraum zu sammeln oder dem Fachmann geläufige Speichelsammelvorrichtungen zum Sammeln der Speichelprobe zu verwenden.

Das erfindungsgemäße Probenmaterial Speichel und die einfache Art der Probengewinnung ermöglicht im Gegensatz zu den bisher in der Influenza-Diagnostik bekannten Probenmaterialien Nasen-/Rachenabstrich und durch Nasenspülung gewonnene Flüssigkeit eine standardisierte Probengewinnung von ungeschulten Personen bzw. dem Patienten selbst und trägt damit zu einer höheren diagnostischen Sicherheit der Influenza A/B-Viren-Teste bei. Zudem ist Speichel als Probenmaterial homogener als die anderen, bislang verwendeten Materialien, was ebenfalls zur diagnostischen Sicherheit beiträgt.

Im erfindungsgemäßen Verfahren wird zunächst eine Speichelprobe gewonnen. Beispielsweise kann dies - wie oben bereits beschrieben - durch Spucken in ein Gefäß, Aufsaugen einer Speichelprobe mittels eines saugfähigen Materials, beispielsweise eines Baumwolltupfers oder ähnlichem, im Mundraum oder unter Benutzung eines herkömmlichen Speichelgewinnungsdevices erfolgen.

Je nach anzuwendender Nachweismethode (immunologischer Nachweis oder Nachweis über entsprechende Nukleinsäuren) wird die Speichelprobe anschließend für die Nachweisreaktion aufbereitet:

Für den immunologischen Nachweis wird aus der Speichelprobe beispielsweise das virale Nukleoprotein von Influenza A/B-Viren durch Lysereagenzien freigesetzt. Solche Reagenzien sind dem Fachmann bekannt und können beispielsweise als wirksame Bestandteile für die Lyse Salze oder Netzmittel enthalten. Lysereagenz zum Nachweis von Influenza-Viren enthält vorzugsweise ein Netzmittel (beispielsweise hat sich Triton^{®} X 100, Tween^{®} 20 oder beta-Octylglycopyranosid bewährt), ein mildes Reduktionsmittel (bsp. N-Acetyl-L-Cystein oder DTT = Dithiothreitol), physiologischges Kochsalz (d.h. 0.9 Gew% NaCl in 20-50mM Phosphatpuffer), ein Konservierungsmittel (bspw. 0,09 Gew% NaN3) und gegebenenfalls ein Protein zur Verminderung der unspezifischen Bindung (bsp. RSA = Rinderserumalbumin oder RPLA = Rinderplasmaalbumin). Der immunologische Nachweis kann außer dem viralen Nukleoprotein beispielsweise das virale Matrixprotein oder die viralen Polymerasen nachweisen. Möglich ist auch der Nachweis des Hämagglutinins oder der Neuraminidase der Influenza A/B-Viren, wozu dann - da diese Virusbestandteile auf der Virusoberfläche sitzen - keine Lyse erforderlich ist.

Für den Nachweis über eine Nukleinsäure wird beispielsweise virale Nukleinsäure (RNA) gewonnen, gereinigt und entsprechend in Gegenwart der für den Nachweis erforderlichen Primer amplifiziert, vorzugsweise mittels der Reversed Transkriptase-Polymerasekettenreaktion (RT-PCR). Diese Schritte sind dem Fachmann bekannt, ebenso wie Nukleinsäurenachweise ohne vorherige Amplifikation.

Der Nachweis der Influenza A/B-Viren in der Speichelprobe erfolgt nach an sich bekannten Verfahren wie in Anspruch 1 oder 6 ausgeführt.

Beim immunologischen Nachweis beispielsweise des viralen Nukleoproteins kann ein Sandwichkomplex mit entsprechend markierten Antikörpern gebildet und nachgewiesen werden. Selbstverständlich sind jedoch auch kompetitive Nachweisformate möglich. Obwohl der Nachweis mittels eines visuell auszuwertenden, immunochromatographischen Teststreifens als Schnelltest bevorzugt ist, kann der Nachweis über alle üblichen immunologischen Verfahren, beispielsweise ELISA, Agglutinationstests, turbidimetrische Tests etc. erfolgen.

Beim Nachweis der Influenza A1B-Viren über Nukleinsäuren wie RNA wird bevorzugt das Produkt der RT-PCR mittels eines markierten Primers markiert und die Markierung des Primers (z. B. ein Enzym- oder Fluoreszenzlabel) je nach Natur der Markierung (Label) detektiert.

### Die Erfindung wird durch das nachfolgende Beispiel näher erläutert:

### Beispiel

### 1. Nachweis einer Influenza-Virus-Infektion aus Speichel als Probenmaterial

Zum Beleg der Eignung von Speichel als Probenmaterial zum Nachweis von Influenza-Viren wurden bei 10 Personen mit Verdacht auf Influenza und bei 4 Personen ohne akuten Influenzaverdacht jeweils zwei Rachenabstriche und eine Speichelprobe entnommen. Das aus den Rachenabstrichen gewonnene Probenmaterial wurde zu Vergleichszwecken gesammelt und auf eine Influenza A/B-Viren-Infektion untersucht. Die Ergebnisse dieser Untersuchungen wurden mit den Ergebnissen verglichen, die mit Speichel als Probenmaterial gewonnen wurden. Tabelle 1, die im Anschluß an den experimentellen Teil des Beispiels wiedergegeben ist, zeigt die Vergleichsergebnisse im Überblick.

### 1.1. Probengewinnung

### 1.1.1. Rachenabstriche

Die Rachenabstriche wurden mittels steriler Einwegbaumwollabstrichtupfer der Firma Copan Italia (I-25125 Brescia; Best.-Nr. 167CS01) in der dem Fachmann geläufigen Art und Weise entnommen. Die jeweils zwei von einem Patienten entnommenen Rachenabstriche wurden durch einmaliges Abstreichen des Rachens mit einem Abstrichtupfer mit zwei unmittelbar nebeneinander angeordneten Baumwollkissen erhalten. Dadurch wurde eine weitgehende Vergleichbarkeit der beiden Abstriche gewährleistet.

### 1.1.2. Speichelprobe

Die Speichelprobe wurde von den Patienten selbst gewonnen, indem sie jeweils ca. 0,5 ml spontan gebildeten Speichel in einem kleinen Einwegkunststoffröhrchen mit Schraubverschluß (Best.-Nr. 62.559.001 der Firma Sarstedt) auffingen. Auf die Verwendung von dem Fachmann bekannten Speichelgewinnungsverfahren/-devices (wie etwa die Salivetten der Firma Sarstedt oder das OraSure Specimen Collection Device der Firma Epitope Inc. Beaverton, OR 97008, USA) wurde bewußt verzichtet um zu zeigen, daß ohne vorherige Konzentrierung oder Speichelprozessierung ein Nachweis von Influenza-Viren möglich ist.

### 1.2. Nachweis von Influenza A/B-Viren aus Rachenabstrichen (nur zum Vergleich)

### 1.2.1. Nachweis aus Rachenabstrichen mittels Zellkultur

Jeweils der erste Abstrichtupfer wurde unmittelbar nach der Entnahme in ein Röhrchen mit 1,5 ml Influenza-Virus-Transportmedium der Firma Virotest (Rosenbergstr. 85, D-70193 Stuttgart, Cat.-No. 0500300) überführt und auf MDCK-Zellen (Madine-Darby Canine Kidney-Zellen) kultiviert. Die Kultivierung der Proben erfolgte gemäß dem in der Literatur beschriebenen Verfahren( Mikrobiologische Diagnostik, Georg Thieme Verlag, Stuttgart, New York, 1992, Herausgeber Friedrich Burkhardt, Seite 371). Die Ergebnisse sind in Tabelle 1 wiedergegeben, wobei "+" einen positiven Befund und "-" einen negativen Befund symbolisiert.

### 1.2.2. Nachweis aus Rachenabstrich mittels eines immunologischen Schnelltests

Der jeweils zweite Abstrichtupfer wurde unmittelbar nach der Entnahme der Probe aus dem Rachenraum des Patienten mittels eines immunologischen Antigenschnelltestes auf Anwesenheit von Influenza-Viren (Influenza A/B-Rapid Test der Firma Roche Diagnostics GmbH, Mannheim, Deutschland, Kat.-No. 2 158 663) geprüft. Der dabei verwendete Schnelltest entspricht im Wesentlichen dem als Beispiel 2 in EP-A 0 926 498 beschriebenen immunologischen Schnelltest. Auf dieses Dokument wird hierzu ausdrücklich Bezug genommen.

Neben der visuellen Bewertung der Teststreifen wurde die Intensität der Nachweislinie nach beendeter Chromatographie mittels eines remissionsphotometrischen Meßgerätes (Ringbeleuchtung mittels 24 grüner LEDs der Wellenlänge 555 nm und CCD-Kamera mit Objektiv) quantitativ vermessen. Dabei wurde die Intensität des Nachweisstrichsignals in Prozenten der Remission (% Rem.; bezogen auf einen "weißen" Bezirk des Teststreifens, dem die Remission 100 % zugeordnet wurde) untersucht: Remissionswerte ab 98,5 % werden vom Benutzer als negatives Signal erkannt; Remissionswerte zwischen 96 % und 98,5 % werden als schwach positives Signal erkannt; Remissionswerte von weniger als 96 % werden als eindeutig positives Signal erkannt. Die Ergebnisse sind in Tabelle 1 wiedergegeben, wobei "+" ein positives Signal, "(+)" ein schwach positives Signal, und "-" ein negatives Signal bedeutet.

### 1.3. Nachweis von Influenza A/B-Viren aus Speichel

### 1.3.1. Nachweis von viraler Nucleinsäure im Speichel mittels RT-PCR

Die Speichelproben wurden mittels RT-PCR (Reversed Transkriptase-Polymerase Chain Reaction) auf Anwesenheit viraler Influenza-Nucleinsäure (hier: Ribonukleinsäure, RNA) geprüft. Der Analysengang setzte sich aus den drei in der Nucleinsäurediagnostik üblichen Teilschritten Probenvorbereitung (zur Abtrennung von Inhibitoren), Amplifikation und Nachweis der Nucleinsäure zusammen (vgl. nachfolgend unter 1.3.1.a bis 1.3.1.c). Dabei wurde wie folgt vorgegangen:

### 1.3.1.a Probenvorbereitung

Zur Isolierung der viralen Influenza-RNA wurde der unter der Best.-Nr. 1 858 882 von Roche Molecular Biochemicals (Sandhofer Straße 116 in D-68305 Mannheim/Deutschland) kommerziell erhältliche "High Pure Viral RNA Kit" verwendet. Die Durchführung erfolgte gemäß dem in der Produktbeschreibung aufgeführten Standard-Protokoll, indem zunächst jeweils 200 µl Speichel pro Reaktionsgefäß (Filtertube) in Anwesenheit von Bindepuffer an das Glasvlies des Filtertubes gebunden wurde und nach Entfernen evtl. anwesender Inhibitoren durch zwei Waschschritte die virale Nucleinsäure in 200 µl Elutionspuffer eluiert wurde. Dabei wurde darauf geachtet, dass das Probenvolumen vor (Speichel) und nach (Eluat) der Probenvorbereitung identisch war, so dass keine Konzentrierung der Influenza-Viren stattgefunden hat. Natürlich ist es erfindungsgemäß auch möglich, den Elutionsschritt mit weniger als der der Speichelmenge entsprechenden Elutionspuffermenge durchzuführen und so die Influenza-Viren im Eluat aufzukonzentrieren bzw. anzureichern.

### 1.3.1.b RT-PCR

Sämtliche Reagenzien wurden, soweit nicht anders aufgelistet, über Roche Molecular Biochemicals (s. o.) bezogen.

Das Ansatzvolumen pro PCR-Reaktionsgefäß betrug 50 µl. Darin waren 10 µl Probenvolumen (Eluat aus der Probenvorbereitung) sowie 10 µl Bicine-Puffer (5 x RT-PCR-Puffer) enthalten. Die weiteren Komponenten des Mastermixes waren in folgender Endkonzentration enthalten: 2,5 mmolar Mangan-acetat, jeweils 0,2 mmolar dATP (2'-Desoxy-adenosin-5'-triphosphat), dCTP (2'-Desoxy-cytidin-5'-triphosphat), dGTP (2'-Desoxy-guanosin-5'-triphosphat) und dUTP (2'-Desoxy-uridin-5-triphosphat) sowie 0,05 mmolar dTTP (2'-Desoxy-thymidin-5'-triphosphat); 0,01 U/µl UNG (Uracil-DNA-Glycosylase); 0,2 U/µl Tth-Polymerase aus *Thermus thermophilus* HB8; 0,8 U/µl RNase-Inhibitor sowie jeweils 1,0 µmolar Forward- und Reverse Primer.

Die Sequenz der Primer wurde der Literatur entnommen (James C. Donofrio et al., Detection of Influenza A and B in Respiratory Secretions with the PCR, 1992, PCR Methods and Applications 1, Seite 263-268). Die Primer sind typspezifisch für Influenza A und weisen ein 212 Basenpaar langes hoch konserviertes Gensegment (Position 101 bis 312) des Matrixgens nach. Der Reverse Primer war zum anschließenden Nachweis des Amplifikationsproduktes in der Mikrotiterplatte am 5'-Ende Biotin-markiert. Typspezifische Primer für Influenza B sind ebenfalls in der Literatur bekannt (james C. Donofrio et al., Detection of Influenza A and B in Respiratory Secretions with the PCR, 1992, PCR Methods and Applications 1, Seite 263-268).

Die Amplifikation des Mastermixes erfolgte in einem Perkin Elmer 9600 Thermocycler mit dem nachfolgenden Temperaturprofil: 20 Minuten Raumtemperatur nach UNG-Zugabe + 45 Minuten 60° C + 2 Minuten 94° C + 10 Zyklen (30 Sekunden 94° C + 60 Sekunden 50° C + 90 Sekunden 68° C) + 35 Zyklen (30 Sekunden 94° C + 60 Sekunden 60° C + 90 Sekunden 68° C) + 7 Minuten 68° C.

### 1.3.1.c Hybridisierung und Detektion

Der Nachweis der aus der mittels RT-PCR amplifizierten Nucleinsäure erfolgte unter Verwendung des PCR ELISA (DIG Detection) der Firma Roche Molecular Biochemicals Cat.-No. 1636111. Mit Ausnahme der Pipettiervolumina wurden sämtliche Schritte gemäß Beipackzettel ausgeführt. 10 µl des Amplifikationsproduktes wurden mit 20 µl Denaturierungslösung in einem 1,5 ml Reaktionsgefäß gemischt und für 10 Minuten bei Raumtemperatur inkubiert. Danach wurden 250 µl einer Digoxigenin-markierten Hybridisierungssonde zugegeben. Die Konzentration der Hybridisierungssonde betrug 70 ng/ml.

Die Sequenz der Hybridisierungssonde wurde der Literatur entnommen (James C. Donofrio et al., Detection of Influenza A and B in Respiratory Secretions with the PCR, 1992, PCR Methods and Applications 1, Seite 263-268). Die Sonde ist gegen die Position 177 - 205 des Matrixgenes (Segment 7) von Influenza A gerichtet. Entsprechende Sonden für Influenza B sind ebenfalls in der Literatur bekannt (James C. Donofrio et al., Detection of Influenza A and B in Respiratory Secretions with the PCR, 1992, PCR Methods and Applications 1, Seite 263-268).

200 µl des Reaktionsgemisches wurden in eine Vertiefung ("well") einer mit Streptavidin beschichteten Mikrotiterplatte überführt und auf einem Schüttler für 1 Stunde bei 37° C inkubiert. Nach Bindung des Hybridisierungsproduktes an das Streptavidin der Mikrotiterplattenwand wird der Inhalt des wells abgesaugt und 3 mal mit jeweils 300 µl Waschlösung gewaschen. Danach wurden 200 µl Anti-Digoxigenin-Peroxidase-Substrat (Anti-DIG-POD-Konjugat) in das well gegeben und die Lösung für 30 Minuten bei 37° C auf einem Schüttler inkubiert. Nach Bindung des POD-Konjugates wurde der Inhalt des wells abgesaugt, 3 mal mit 300 µl Waschlösung gewaschen und anschließend 200 µl 2,2'-Azino-di-[3-ethylbenzthiazolin sulfonat (6)] Diammoniumsalz-Substrat (ABTS-Substrat) zugegeben. Nach abschließender Inkubation für 30 Minuten bei 37° C wurde die Farbentwicklung bei 405 nm an einem Mikrotiterplattenreader gemessen.

Bei jeder PCR-Bestimmung von Speichelproben wurden parallel mehrere Negativkontrollen (DEMC-Wasser (Art. Nr. 16-001Y "Accu Gene Water, molecular biology grade, autoclaved" des Herstellers Bio Whittaker Europe S.p.r.l. (Parc Industriel de Petit-Rechain, B-4800 Verviers, Belgium) zum Ausschluß falsch positiver Werte durch Kontamination) sowie mehrere Positivkontrollen (Verdünnungsreihen von influenzahaltigen MDCK-Kulturüberständen) mittels Probenvorbereitung und RT-PCR mitbestimmt. Zusätzlich wurde zur Kontrolle der Detektionsreagenzien auf der Mikrotiterplatte eine kitinterne Positivkontrolle mitgeführt.

Die Extinktionswerte der mitgeführten Negativkontrollen in der Mikrotiterplatte lagen üblicherweise bei 100-150 mE. Die Werte der mitgeführten kitinternen Positivkontrolle lagen üblicheweise bei 1000-1500 mE.

Als Positivsignal für Patientenproben wurden Extinktionswerte ≥ 300 mE definiert, was einem Signal größer dem 2fachen Leerwert/Nullwert entspricht. Die Ergebnisse finden sich in Tabelle 1, wobei "+" einen positiven Befund und "-" einen negativen Befund symbolisiert.

### 1.3.2. Nachweis eines viralen Antigens im Speichel mittels eines immunologischen Schnelltestes

Zum Nachweis der Influenza A/B-Viren aus Speichel wurde der Influenza A/B-Rapid Test der Firma Roche Diagnostics Cat.-No. 2 158 663 verwendet. Der dabei verwendete Schnelltest entspricht im Wesentlichen dem als Beispiel 2 in EP-A 0 926 498 beschriebenen immunologischen Schnelltest. Auf dieses Dokument wird hierzu ausdrücklich Bezug genommen.

Der Test wird normalerweise dazu verwendet, das typspezifische virale Nucleoprotein aus Rachenabstrichen mittels eines immunologischen Chromatographieteststreifens nachzuweisen. Der Test differenziert dabei nicht zwischen Influenza A- und Influenza B-Viren.

Um eine gute Chromatographie des Teststreifens mit Speichel als Probenmaterial zu gewährleisten, wurde ein spezieller Lyse/Elutionspuffer verwendet, der nicht Bestandteil der Testpackung ist. Der spezielle Lyse/Elutionspuffer setzte sich wie folgt zusammen:
0,9 Gew.-% NaCl, 2 mM KH₂PO₄, 10 mM Na₂HPO₄, 0,095 Gew.-% NaN₃, 10 mM EDTA,
1,5 Gew.-% Rinderserumalbumin (RSA), 1,5 Gew.-% Triton^{®} X-100.

Der Test wurde wie folgt durchgeführt:

Anstatt wie im Beipackzettel beschrieben zunächst einen Rachenabstrichtupfer als Patientenprobe mit 3 Portionen (= 800 µl) des im Testkit enthaltenen Lyse/Elutionspuffers zu eluieren, wurden 300 µl Speichel und 500 µl des speziellen Lyse/Elutionspuffers in ein Reaktionsgefäß gegeben und gemischt.

Die weitere Testdurchführung erfolgte gemäß den im Beipackzettel beschriebenen Schritten. Dies beinhaltete zunächst die Zugabe von 2 Tropfen Antikörperlösung 1 (enthält biotinylierte monoklonale Antikörper gegen Nucleoprotein A und Nucleoprotein B), die Zugabe von 2 Tropfen Antikörperlösung 2 (enthält digoxigenylierte monoklonale Antikörper gegen Nucleoprotein A und Nucleoprotein B) sowie die abschließende Chromatographie dieses Reaktionsgemisches über einen Teststreifen.

Wie aus EP-A 0 926 498 bekannt ist enthält der Teststreifen ein Konjugatvlies, das mit einem in Probenflüssigkeit ablösbarem Goldkonjugat reversibel imprägniert ist. Die Goldpartikel sind dabei adsorptiv mit einem monoklonalen Antikörper gegen Digoxigenin beladen.

In einer weiteren Zone des Teststreifens in Chromatographierichtung flußabwärts befindet sich eine Nitrocellulosemembran, auf der Polystreptavidin als Nachweislinie und ein polyklonaler Antikörper PAK<Maus Fcγ>S-IgG als Kontroll-Linie irreversibel imprägniert sind.

Der in Anwesenheit des Analyten im Reaktionsgefäß gebildete Sandwichkomplex aus biotinyliertem Antikörper/Nucleoprotein/digoxigenyliertem Antikörper chromatographiert über den Teststreifen, bindet nach Anlösen des Goldkonjugates dieses über den Anti-Digoxigenin-Antikörper an den digoxigeninmarkierten Anti-Influenza-Antikörper des Sandwich-Komplexes und wird anschließend am Polystreptavidinstrich über den biotinmarkierten Anti-Inffuenza-Antikörper des Sandwich-Komplexes an der Nitrocellulosemembran abgefangen. Dabei wird ein roter Strich auf der Nitrocellulosemembran sichtbar, der ein positives Testsignal darstellt.

Überschüssiges Goldkonjugat chromatographiert flußabwärts und wird am Kontrollstrich der Nitrocellulosemembran als weitere, sichtbare rote Linie mittels PAK<Maus Fcγ>S-IgG abgefangen.

Neben der visuellen Bewertung der Teststreifen wurde die Intensität der Nachweislinie nach beendeter Chromatographie mittels eines remissionsphotometrischen Meßgerätes (Ringbeleuchtung mittels 24 grüner LED's der Wellenlänge 555 nm und CCD-Kamera mit Objektiv) quantitativ vermessen. Dabei wurde die Intensität des Nachweisstrichsignals in Prozenten der Remission (% Rem.; bezogen auf einen "weißen" Bezirk des Teststreifens, dem die Remission 100 % zugeordnet wurde) untersucht: Remissionswerte ab 98,5 % werden vom Benutzer als negatives Signal erkannt; Remissionswerte zwischen 96 % und 98,5 % werden als schwach positives Signal erkannt; Remissionswerte von weniger als 96 % werden als eindeutig positives Signal erkannt. Die Ergebnisse sind in Tabelle 1 wiedergegeben, wobei "+" ein positives Signal, "(+)" ein schwach positives Signal und "-" ein negatives Signal darstellt.

### 1.4. Ergebnisse

Nachfolgend sind einige mit Patientenproben erhaltene Ergebnisse beispielhaft aufgeführt (Tabelle 1).

Die aufgeführten Ergebnisse beziehen sich ausschließlich auf den Nachweis von Influenza A-Viren, da zum Zeitpunkt der Probennahme ausschließlich Influenza A vorherrschte und keine Patientenproben mit Influenza B gefunden wurden. Den Experten ist bekannt, daß Influenza B im Gegensatz zu Influenza A nicht in jeder Wintersaison zirkuliert und daß selbst bei einem gemeinsamen Auftreten von Influenza A/B-Viren in einer Wintersaison Influenza B immer mit einer deutlich niedrigeren Prävalenz vertreten ist.

Es soll hier nicht unerwähnt bleiben, dass künstlich hergestellte Influenza B-positive Speichelproben (gepoolte Speichelproben, die mit Kulturüberstand aus Influenza B-Viren aufgestockt ("gespiket") waren) sowohl im Nukleinsäuretest als auch im immunologischen Test ebenfalls zum Nachweis von Influenza-Viren aus Speichel geeignet waren. Die aufgeführten Ergebnisse stellen daher keine Limitation im Sinne der Erfindung zum separaten oder gemeinsamen Nachweis von Influenza B-Viren neben Influenza A-Viren aus Speichel dar. Vielmehr ist Speichel als Probenmaterial zum Nachweis von Influenza A/B-Viren geeignet.

**Tabelle 1**

| Person Nr.^{A} | Rachenabstrichproben (nur zum Vergleich) | | | Speichelproben | | |
|---|---|---|---|---|---|---|
| | Kultur^{B} | immunologischer Schnelltest | | PCR-Ergebnis^{E} | immunologischer Schnelltest | |
| | | visuell^{C} | photometrisch^{D} | | visuell^{C} | photometrisch^{D} |
| 1 | + | + | + | + | + | + |
| 2 | + | (+) | (+) | + | + | + |
| 3 | + | (+) | (+) | + | + | + |
| 4 | + | + | + | + | + | + |
| 5 | + | + | + | + | + | + |
| 6 | + | (+) | (+) | + | + | + |
| 7 | + | - | - | + | (+) | (+) |
| 8 | + | - | - | + | (+) | (+) |
| 9 | - | - | - | - | - | - |
| 10 | - | - | - | - | - | - |
| 11 | - | - | - | - | - | - |
| 12 | - | - | - | - | - | - |
| 13 | - | - | - | - | - | - |
| 14 | - | - | - | - | - | - |

| | | | | | | |
|---|---|---|---|---|---|---|
| A: Bei den Personen Nr. 1-10 handelte es sich um Personen mit influenzaartiger Symptomatik. Bei den Personen Nr. 11-14 handelte es sich um Personen ohne akute respiratorische Symptomatik. B: Kultivierung auf MDCK-Zellen (vgl. unter 1.2.1. oben); + positiv; - negativ C: Schnelltestdurchführung wie unter 1.2.2. und 1.3.2. beschrieben (siehe oben); + positiv; (+) schwach positiv; - negativ D: Quantitative Bestimmung der Intensität des Nachweisstriches mittels eines remissionsphotometrischen Meßgerätes (vgl. unter 1.2.2. und 1.3.2. oben); wiedergegeben sind hier nur die relativen Ergebnisse (+ positiv; (+) schwach positiv; - negativ), die sich gemäß der im Text angegebenen Skala ergeben. E: Gemessen wurden die Extinktionswerte der Mikrotiterplatte bei 405 nm (vgl. unter 1.3.1. oben); wiedergegeben sind hier nur die relativen Ergebnisse (+ positiv; - negativ), die sich gemäß der im Text angegebenen Skala ergeben. | | | | | | |

Aus den Ergebnissen, die in Tabelle 1 wiedergegeben sind, wird folgendes sichtbar:
- Nicht alle aufgrund der klinischen Symptomatik als Influenzapatienten eingestuften Personen (Nr. 1-10) waren tatsächlich Influenza-positiv. Person Nr. 9 und 10 waren sowohl in der Kultur aus Rachenabstrich, als auch in der PCR aus Speichel als auch mit dem Schnelltest aus Speichel und Rachenabstrich negativ. Dies verdeutlicht die einleitend gemachte und in Expertenkreisen bekannte Aussage, daß eine definitive Diagnose auf Influenza ausschließlich aufgrund der klinischen Symptomatik nicht möglich ist.
- Die Rachenabstriche und Speichelproben von asymptomatischen Personen (Nr. 11- 14) waren mit allen aufgelisteten Testmethoden negativ und verdeutlichen damit die klinische Spezifität dieser Methoden.
- Von den 8 über Rachenabstriche mittels Zellkultur Influenza A-positiv gefundenen Personen (Nr. 1-8; sog. kulturpositive Patienten) wurden mit dem immunologischen Schnelltest aus Rachenabstrichen 6 Personen ebenfalls als positiv diagnostiziert. Dies verdeutlicht, daß die klinische Sensitivität dieser bisher verfügbaren Schnellteste aus Abstrichen als Probenmaterial unter der zur Zeit als Goldstandard anerkannten Zellkultur liegt (klinische Sensitivitäten verschiedener immunologischer Schnellteste gemäß Beipackzettel der jeweiligen Hersteller: Quidel 73 % für Nasenabstrich, Roche 70 % für Rachenabstrich, Biostar 62 % für Rachenabstrich und 83 % für Nasopharyngealabstrich).
- Hingegen wurden die zugehörigen Speichelproben aller 8 kulturpositiven Patienten mit dem immunologischen Schnelltest positiv gefunden.
- Desweiteren geht aus dem Vergleich der Remissionswerte (in der Tabelle nicht gezeigt) hervor, daß die Nachweisstrichintensitäten auf dem immunologischen Schnellteststreifen mit Speichelprobe etwas intensiver waren als die zugehörigen Nachweisstrichintensitäten bei Verwendung von Rachenabstrichen.
- Die Schnelltestergebnisse aus Speichel decken sich dabei mit den zugehörigen PCR-Ergebnissen aus Speichel und belegen damit, daß aus Speichel als Probenmaterial auch ohne vorherige Anreicherung bei der Probengewinnung Influenza A/B-Viren zuverlässig nachgewiesen werden können.

## Patentansprüche

1. Verfahren zum Nachweis einer Infektion mit dem Influenza A- und/oder Influenza B-Virus umfassend die Schritte:
i) Gewinnung einer Speichelprobe;
ii) Vorbereitung der Speichelprobe für den Nachweis; und
iii) Nachweis des Influenza A- und/oder Influenza B-Virus in der Speichelprobe **dadurch gekennzeichnet, dass** die Speichelprobe, die in Schritt i) gewonnen wird, spontan gebildeter Speichel ist, wobei bei der Probennahme der Speichelprobe keine Anreicherung der Viren in der Probe erfolgt, sondern nur der spontan im Mundraum befindliche Speichel gesammelt wird.

2. Verfahren gemäß Anspruch 1, **dadurch gekennzeichnet, dass** die Speichelprobe in Schritt i) mit einem Speichelgewinnungsdevice ohne virusspezifische Anreicherung gewonnen wird.

3. Verfahren gemäß einem der Ansprüche 1 bis 2, **dadurch gekennzeichnet, dass** in Schritt ii) aus der Speichelprobe virale Influenza A- und/ oder Influenza B-RNA gewonnen wird, diese mittels RT-PCR amplifiziert und in Schritt iii) nachgewiesen wird.

4. Verfahren gemäß einem der Ansprüche 1 bis 2, **dadurch gekennzeichnet, dass** die Speichelprobe in Schritt iii) immunologisch auf Vorhandensein von influenza A- und/oder Influenza B-Viren untersucht wird.

5. Verfahren gemäß Anspruch 4, **dadurch gekennzeichnet, dass** die Speichelprobe in Schritt ii) mit einem Lysepuffer behandelt wird, so dass virales Nukleoprotein des Influenza A- und/ oder Influenza B-Virus freigesetzt wird und dieses Nukleoprotein in Schritt iii) immunologisch nachgewiesen wird.

6. Verwendung von Speichel als Probenmaterial zum Nachweis einer Infektion mit dem Influenza A- und/oder Influenza B-Virus **dadurch gekennzeichnet, dass** der Speichel spontan gebildeter Speichel ist, wobei bei der Probennahme der Speichelprobe keine Anreicherung der Viren in der Probe erfolgt, sondern nur der spontan im Mundraum befindliche Speichel gesammelt wird und der Nachweis der in der Probe befindlichen Influenza A und oder Influenza B Viren erfolgt.

7. Verwendung von Speichel gemäß Anspruch 6, **dadurch gekennzeichnet, dass** die Speichelprobe mit einem Speichelgewinnungsdevice ohne virusspezifische Anreicherung gewonnen wird.

8. Verwendung von Speichel gemäß einem der Ansprüche 6 bis 7, **dadurch gekennzeichnet, dass** der Nachweis einer Infektion mit dem Influenza A- und/oder Influenza B-Virus mittels eines immunologischen Tests erfolgt.

9. Verwendung von Speichel gemäß einem der Ansprüche 6 bis 7, **dadurch gekennzeichnet, dass** der Nachweis einer Infektion mit dem Influenza A- und/oder Influenza B-Virus mittels eines Nukleinsäurenachweises erfolgt.

## Claims

1. Method for the detection of an infection with the influenza A and/or influenza B virus comprising the steps:
i) obtaining a saliva sample,
ii) preparing the saliva sample for the detection and
iii) detecting the influenza A and/or influenza B virus in the saliva sample,
**characterized in that** the saliva sample which is obtained in step i) is spontaneously formed saliva, wherein no enrichment of the viruses in the sample takes place when the saliva sample is taken but rather only the saliva which is spontaneously present in the mouth space is collected.

2. Method according to claim 1, **characterized in that** the saliva sample in step i) is obtained with a saliva collection device without virus-specific enrichment.

3. Method according to one of the claims 1 to 2, **characterized in that** viral influenza A and/or influenza B RNA is isolated from the saliva sample in step ii), amplified by RT-PCR and detected in step iii).

4. Method according to one of the claims 1 to 2, **characterized in that** in step iii) the saliva sample is examined immunologically for the presence of influenza A and/or influenza B viruses.

5. Method according to claim 4, **characterized in that** the saliva sample is treated in step ii) with a lysis buffer such that viral nucleoprotein of the influenza A and/or influenza B virus is released and this nucleoprotein is immunologically detected in step iii).

6. Use of saliva as the sample material for the detection of an infection with the influenza A and/or influenza B virus, **characterized in that** the saliva is spontaneously formed saliva, wherein no enrichment of the viruses in the sample takes place when the saliva sample is taken but rather only the saliva which is spontaneously present in the mouth space is collected and the influenza A and/or influenza B viruses present in the sample are detected.

7. Use of saliva according to claim 6, **characterized in that** the saliva sample is collected with a saliva collecting device without virus-specific enrichment.

8. Use of saliva according to one of the claims 6 to 7, **characterized in that** an infection with the influenza A and/or influenza B virus is detected by means of an immunological test.

9. Use of saliva according to one of the claims 6 to 7, **characterized in that** an infection with the influenza A and/or influenza B virus is detected by means of a nucleic acid detection.

## Revendications

1. Procédé pour le décèlement d'une infection par le virus de la grippe A et/ou le virus de la grippe B comprenant les étapes:
i) de récupération d'un d'échantillon de salive ;
ii) de préparation de l'échantillon de salive pour le décèlement ; et
iii) de décèlement du virus de la grippe A et/ou du virus de la grippe B dans l'échantillon de salive,
**caractérisé en ce que** l'échantillon de salive qui est récupéré à l'étape i) est de la salive qui s'est formée spontanément, aucun enrichissement des virus dans l'échantillon n'ayant lieu lors du prélèvement de l'échantillon de salive, seule la salive se trouvant spontanément dans la cavité buccale étant récoltée.

2. Procédé selon la revendication 1, **caractérisé en ce qu'**on récupère l'échantillon de salive à l'étape i) avec un dispositif de récupération de salive en l'absence d'un enrichissement viral spécifique.

3. Procédé selon l'une quelconque des revendications 1 à 2, **caractérisé en ce qu'**on récupère l'ARN viral de la grippe A et/ou de la grippe B à partir de l'échantillon de salive à l'étape ii), on amplifie l'ARN en question par RT-PCR et on procède au décèlement à l'étape iii).

4. Procédé selon l'une quelconque des revendications 1 à 2, **caractérisé en ce que** l'échantillon de salive est soumis à un examen immunologique à l'étape iii) pour la détection de la présence du virus de la grippe A et/ou du virus de la grippe B.

5. Procédé selon la revendication 4, **caractérisé en ce que** l'échantillon de salive est soumis à un traitement avec un tampon de lyse à l'étape ii), de façon à libérer la nucléoprotéine virale du virus de la grippe A et/ou du virus de la grippe B, cette nucléoprotéine faisant l'objet d'un décèlement immunologique à l'étape iii).

6. Utilisation de salive à titre de matière échantillon pour le décèlement d'une infection par le virus de la grippe A et/ou le virus de la grippe B, **caractérisée en ce que** la salive est de la salive qui s'est formée spontanément, aucun enrichissement des virus dans l'échantillon n'ayant lieu lors du prélèvement de l'échantillon de salive, seule la salive se trouvant spontanément dans la cavité buccale étant récoltée et le virus de la grippe A et/ou le virus de la grippe B se trouvant dans l'échantillon étant décelé(s).

7. Utilisation de salive selon la revendication 6, **caractérisée en ce que** l'échantillon de salive est récupéré avec un dispositif de récupération de salive en l'absence d'un enrichissement viral spécifique.

8. Utilisation de salive selon l'une quelconque des revendications 6 à 7, **caractérisée en ce que** le décèlement d'une infection par le virus de la grippe A et/ou par le virus de la grippe B a lieu au moyen d'un test immunologique.

9. Utilisation de salive selon l'une quelconque des revendications 6 à 7, **caractérisée en ce que** le décèlement d'une infection par le virus de la grippe A et/ou par le virus de la grippe B a lieu au moyen d'un décèlement d'acide nucléique.
